(19) **Europäisches Patentamt**
**European Patent Office**
**Office européen des brevets**

(11) **EP 3 833 307 B1**

(12) **EUROPEAN PATENT SPECIFICATION**

(45) Date of publication and mention
of the grant of the patent:
**15.06.2022 Bulletin 2022/24**

(21) Application number: **19759438.5**

(22) Date of filing: **01.08.2019**

(51) International Patent Classification (IPC):
*A61F 2/58* (2006.01)  *B25J 17/00* (2006.01)
*B25J 17/02* (2006.01)  *A61F 2/60* (2006.01)
*A61F 2/50* (2006.01)

(52) Cooperative Patent Classification (CPC):
**A61F 2/585; A61F 2/58; A61F 2/604;**
**B25J 17/0233; B25J 17/0241;** A61F 2002/5007;
A61F 2002/5018; A61F 2002/5038;
A61F 2002/5039; A61F 2002/5072

(86) International application number:
**PCT/IB2019/056570**

(87) International publication number:
**WO 2020/031035 (13.02.2020 Gazette 2020/07)**

(54) **MODULE OF FLEXION-EXTENSION FOR AN ARTIFICIAL ARTICULATION**

MODUL FÜR BEUGUNG-STRECKUNG FÜR EIN KÜNSTLICHES GELENK

MODULE DE FLEXION-EXTENSION POUR UNE ARTICULATION ARTIFICIELLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.08.2018 IT 201800007933**

(43) Date of publication of application:
**16.06.2021 Bulletin 2021/24**

(73) Proprietors:
• **Università Campus Bio-Medico di Roma
00128 Roma (RM) (IT)**
• **INAIL - Istituto Nazionale per l'Assicurazione
contro gli Infortuni sul Lavoro
00187 Roma (RM) (IT)**

(72) Inventors:
• **CARPINO, Giorgio
80123 Napoli (NA) (IT)**
• **BRAMATO, Laura
00128 Roma (RM) (IT)**
• **ZOLLO, Loredana
82100 Benevento (BN) (IT)**
• **SIMONETTI, Davide
00128 Roma (RM) (IT)**

(74) Representative: **Manna, Sara et al
Società Italiana Brevetti S.p.A.
Piazza di Pietra, 39
00186 Roma (IT)**

(56) References cited:
US-A- 4 994 078   US-A1- 2007 173 955
US-A1- 2008 033 565   US-B1- 6 626 913

**EP 3 833 307 B1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Technical field of the invention**

The present invention refers to a module of flexion-extension for an artificial articulation, as defined in claims 1-7, and further a mechanical joint, an artificial articulation and a prosthesis assembly comprising said module of flexion-extension as defined in claims 8-10, respectively. The disclosure also describes a robotic joint comprising a module of pronation-supination and a module of flexion-extension, each respectively configured to allow the reproduction of a pronation-supination motion and a flexion-extension motion by the articulation itself.

**Background**

In the state of the art, several technical solutions are known which allow the reproduction of human articulations, such as wrists, knees and ankles, by means of active or passive elastic joints. Such joints are designed to couple with prosthetic devices, such as prosthetic hands, legs and feet, as well as with respective prosthetic sockets, i.e. with interface elements between the free end of the amputated limb and the artificial joint or the prosthesis itself.

With particular reference to prosthetic systems for the wrist articulation, these are designed to fulfil two main functions: connecting a hand prosthesis to the forearm and allowing its correct positioning before the execution of a movement.

The human wrist is provided with three degrees of freedom, respectively associated with three separate movements: pronation-supination, flexion-extension and adduction-abduction. Several technological solutions are known in order to reproduce these degrees of freedom, hitherto.

In particular, the degree of freedom whereon the speculative activity historically focused is the one associated with the pronation-supination movement, as it is essential for adjusting the orientation of the hand. Only in recent times several solutions have been developed to further perform flexion-extension and adduction-abduction using an artificial wrist.

Artificial wrist modules of the known type may be grouped based on the implementation mode, which can be active (i.e. provide for the presence of selectively activatable dedicated implementation means) or passive (absence of implementation means).

Referring to the passive implementation mode, a prosthetic wrist module which has an oval shape is known, suitable for fitting to the typical shapes of sockets and prostheses. This module is mounted between socket and prosthetic limb and rotated by the contralateral limb thanks to a friction system. The prosthetic wrist module further comprises rotational mechanisms with adjustable friction to perform the pronation/supination motion.

Furthermore, wrist modules for passive flexion/extension are commercially available, which allow manual positioning in different flexion-extension configurations thanks to friction systems with locking mechanisms. Among the various available models, several of them may achieve a 360° passive rotation, as well as different adjustable flexion/extension angular configurations.

Furthermore, passive pulse modules are known which allow to reproduce all the above three degrees of freedom. Such modules are made with spherical joints, allowing the prosthesis to be pre-positioned, for every degree of freedom, due to retaining a constant friction. In addition, the friction intensity may be adjusted by an action of the user himself.

With regard to active pulse modules, solutions are known which differ by the implemented degrees of freedom, for example single-degree wrist modules and multiple-degree of freedom wrist modules.

In particular, it is worth citing an active wrist module characterized by two degrees of freedom, which allows to operate pronation-supination and flexion-extension by means of two motors in differential configuration: when the two motors rotate in the same direction, the end parts of the differential mechanism rotate in the opposite direction, generating pronation-supination movement. The flexion-extension is instead ensured by the rotation of the motors in the opposite direction.

Furthermore, a pulse module with two degrees of freedom is known, configured to reproduce flexion-extension and adduction-abduction implemented. The structure of this module comprises a spherical joint with increased excursion (between 15°-25°). The above two degrees of freedom are controlled through the use of two separate actuators.

Disadvantageously, although the range of movement which is implemented is very similar to that from a natural wrist, the final size and weight are very high, thus limiting its potential use.

Furthermore, a problem affecting the prosthetic wrist modules known in the state of the art is the high complexity thereof, generally associated with high costs and excessive dimensions of the prostheses.

US2007/173955A1 describes a module of flexion-extension for an artificial wrist joint, which comprises: a base plate configured for connection to a prosthetic arm; a cross element coupled to the base plate, bearing a first axis and a second axis; a prosthetic connection coupled to the cross element, wherein the cross element allows the user to simultaneously flex the prosthetic connection according to two different axes due to the presence of a first and a second torsion springs respectively associated with said axes.

## Summary of the invention

The technical problem posed and solved by the present invention is therefore to provide a module of flexion-extension which allows to overcome the above-mentioned drawbacks with reference to the prior art.

The aforementioned drawbacks are solved by a module of flexion-extension according to claim 1, suitable to be associated with a prosthetic socket and a prosthetic limb, respectively.

Furthermore, the present invention provides a mechanical joint comprising in association the aforementioned module of flexion-extension and a module of pronation-supination according to claim 8, as well as an artificial articulation according to claim 9 and a prosthetic assembly according to claim 10.

Preferred features of the present invention are the subject-matter of the dependent claims.

The present invention provides a passive module of flexion-extension, i.e. which can be devoid of dedicated automatic driving means because it is configured to be operated by an external stress, exerted for example by the user himself.

The external stress is to be such as to cause the module to flex or to extend with respect to a rest configuration. The rest configuration is defined as a configuration wherein an alignment of the prosthetic hand and the socket is realized along the longitudinal development direction of the socket itself.

Furthermore, the stress to realize the flexion/extension is to be of such an intensity as to overcome a contrast force associated with the presence of elastic reaction means. Such reaction means is configured in such a way as to resist to the movement of flexion/extension of the module, in order to return it back to the rest configuration.

The module of flexion-extension further comprises mechanisms apt to transform the movement of flexion/extension, which is expressed in a movement of the module (and thence of the prosthetic hand connected thereto) along a circular trajectory, in a linear movement of stressing means of the aforementioned reaction means.

According to a preferred variant of the invention, such mechanisms comprise a couple of Scoth-Yoke mechanisms, arranged in parallel, each associated with respective elastic reaction means.

A preferred aspect of the invention provides the elastic reaction means as compression springs, and that the stressing means is accordingly configured to compress such springs when a flexion or extension movement of the module is realized.

Preferred variants of the invention provide the module of flexion-extension with a blocking system of the module in predetermined flexion-extension positions, in order to ensure the use further according to a rigid mode.

It should be understood that the same basic mechanism for the module of flexion-extension, suitably re-adjusted, can be further used to implement the flexion-extension of an ankle prosthesis or for a robotic joint applied to rehabilitation or assistance machines.

The present invention further provides a mechanical joint comprising a module of flexion-extension according to the above description and a module of pronation-supination, suitable for reproducing a human wrist joint by the presence of the two moving modules. The module of pronation-supination and the module of flexion-extension are implemented independently from each other, so that the pronation-supination motion and the module of flexion-extension can be separately implemented.

The joint according to the present invention is suitable to reproduce the pronation-supination according to an active mode of implementation, and the flexion-extension according to a passive actuation mode.

According to preferred variants, the module of pronation-supination is implemented by means of a gear motor, which in a preferred variant is comprised into a socket for coupling the robotic joint to the amputated limb. In particular, the gear motor is installed within the socket arranged according to the main longitudinal development direction of the socket itself.

As anticipated, the module of flexion-extension has instead a passive operation, which is delegated to the user himself, for example by using the healthy limb.

Furthermore, preferred variants of the invention provide that the prosthetic wrist can be used as a stand-alone module, which can be integrated with different hand prostheses of a known type. The robotic joint subject-matter of the present invention is further modular and highly customizable, since the features of the module of flexion-extension can be adjusted depending on the features of the elastic reaction means.

Moreover, according to preferred embodiments, the mechanical joint according to the present invention allows to implement a prosthetic wrist articulation having reduced weight and dimensions compared to those of the known type, as well as reliability, strength and limited costs.

Based on the chosen application field, the modularity of the joint according to the present invention allows various elastic behaviours of the joint to be obtained depending on the elastic responding means used, varying the output torsional stiffness thereof.

According to preferred variants, a constant force output feature is obtained by using of variable stiffness springs characterized by an increasing/decreasing helix pitch.

Other advantages, features and methods of use of the present invention will become apparent from the following detailed description of several embodiments, introduced by the way of non-limiting example.

**Short description of the figures**

Reference will be made to the attached figures, wherein:

- Figure 1 shows a schematic block view of a preferred embodiment of an artificial joint according to the present invention;

- Figures 2 to 4 respectively show, in front view, a schematic representation of a preferred embodiment of a module of flexion-extension according to the present invention, in rest position, in a flexed-extended position and in rest position and flexed-extended superimposed - in particular, in Figure 4 the displacements occurred to the various elements of the module passing from one configuration to another are denoted by light grey arrows;

- Figure 5 shows a graph of the trend for the torque T generated by the prosthetic wrist joint according to the present invention, as a function of the rotation angle θ, for different values of the distance R of the axis of the compression springs from the rotation centre of the prosthetic wrist joint;

- Figure 6 shows a front view of a preferred embodiment of a module of flexion-extension according to the present invention, in a rest configuration;

- Figure 7 shows a perspective view of the module of flexion-extension of Figure 6, in a rest configuration;

- Figures 8A to 8C respectively show a side view of the module of flexion-extension of Figure 6 in a flexion, rest and extension configuration;

- Figure 9 shows a perspective view of the module of flexion-extension of Figure 6, in a flexion configuration of 25° with respect to the socket;

- Figures 10A to 10C respectively show a preferred embodiment of a prosthetic forearm assembly according to the present invention, comprising a socket, a prosthetic hand (the exemplary images of the latter prosthetic hand are available on *https://grabcad.com/library/robotic-hand*-22) and a prosthetic wrist joint comprising the module of flexion-extension of Figure 6, in a top, side and bottom view; and

- Figures 11A and 11B respectively show side views of the preferred embodiment of the prosthetic forearm assembly of Figures 10A-10C, in flexion and extension configuration.

The figures above indicated are exclusively intended for illustrative and non-limiting purposes.

**Detailed description of preferred embodiments**

The present invention refers to a mechanical joint suitable to implement a prosthetic wrist articulation, which comprises an innovative module for implementing the wrist movement of flexion-extension. Subsequently, for sake of simplicity, reference will be made to this implementation of the invention for the sole purpose of example, while it should be understood that it may be used to implement further artificial articulations, such as of knee or ankle.

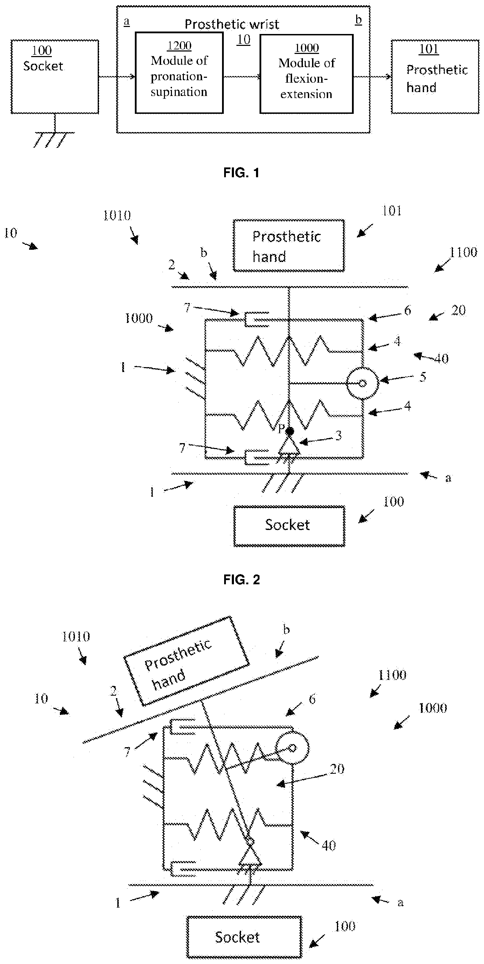

With reference to the diagram of Figure 1, a preferred embodiment of a mechanical joint 1100 for a prosthetic wrist articulation 10 according to the present invention is configured to be coupled to a prosthesis 101, in particular a prosthetic hand, at a proper distal end *b*, and to a prosthetic socket 100 at its own proximal end a.

The joint 10 is configured to perform a movement of the prosthetic hand 101 regarding flexion-extension and pronation-supination with respect to the socket 100, reproducing the typical movement of the human wrist joint.

In particular, the joint 10 according to the present invention is configured to actively implement the pronation-supination movement (hereinafter more briefly pronation-supination), which is fundamental to determine the orientation of the prosthetic hand in space, and in a passive and adaptive way the flexion-extension motion (hereinafter more briefly flexion-extension).

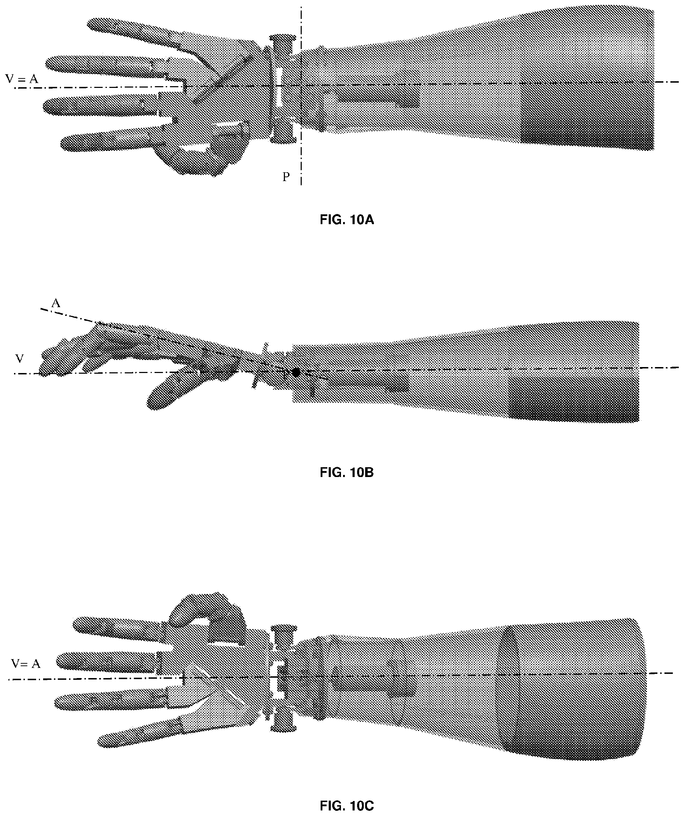

In the present specification, pronation means the position of the hand with the palm facing down when the arm is extended, on the other hand supination means the position of the hand with the palm facing upwards when the arm is extended. As concerns the flexion, this means the movement of the palm approaching the arm, while the extension of the hand means the movement which allows the back of the hand approaching the arm. By way of example, an extension configuration is shown in Figure 10B.

These movements are evaluated with respect to a neutral or rest configuration of the prosthetic hand, shown in Figures 10A and 10C, which corresponds to a position wherein the longitudinal axis V of the socket (or of the patient's forearm the socket is applied thereto) is aligned with the longitudinal axis A of the prosthetic hand, i.e. the longitudinal axis V of the socket and the longitudinal axis A of the hand lie on the same line. In the present specification, both the longitudinal axis of the prosthetic hand and the longitudinal axis of the extension flexion module 1000 are denoted by the literal reference A, which axes for sake of simplicity are assumed to be coincident. In particular, the longitudinal axis A is orthogonal with respect to the distal base 2.

With further reference to the diagram of Figure 1, the mechanical joint 1100 according to the present invention comprises a module of pronation-supination 1000 and a module of flexion-extension 1200.

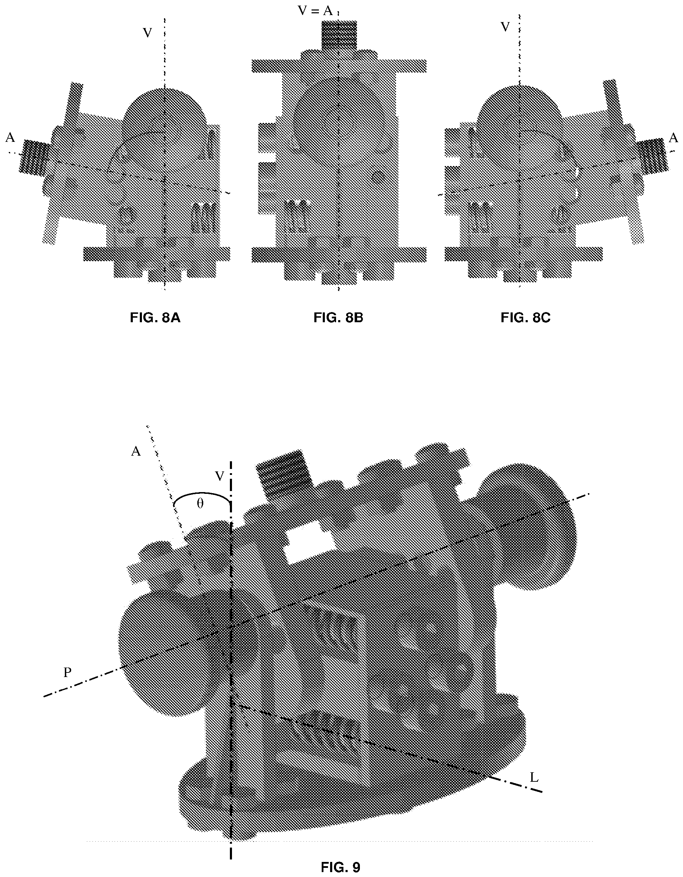

These modules 1000, 1200 are respectively configured to implement the movement of the prosthetic wrist joint 10 preferably in a symmetrical range of motion, which covers most of the range of motion of the wrist in pronation-supination (-85°/+90° about the longitudinal axis V of the prosthetic hand/module of flexion-extension, shown in Figure 9) and in flexion-extension (-80°/+70° about a transverse axis P of the prosthetic hand/module of flexion-extension, shown again in Figure 9).

The module of flexion-extension 1000 and the module of pronation-supination 1200 may mechanically connect to a socket 100 and to a prosthetic hand 101.

In particular, the module of flexion-extension 1000 is preferably configured to connect in series to the module of pronation-supination 1200. In particular, in use, the module of pronation-supination 1200 is respectively connected to the socket 100 and the module of flexion-extension 1000, while the latter module of flexion-extension 1000 is respectively connected to the module of pronation-supination 1200 and to the prosthetic hand 101. Therefore, when the module of pronation-supination 1200 is implemented, even the module of flexion-extension 1000 is liable to pronation/supination motion.

As anticipated, the module of pronation-supination 1200 is implemented by dedicated means, such as for example an actuator.

In order to minimize the overall dimensions and to reduce inertia, the module of pronation-supination 1200 actuator is preferably positioned inside the socket and arranged along its longitudinal development axis, in particular by providing a connection of the gear motor stator to the socket frame. The output shaft of the actuator is preferably connected to the module of flexion-extension 1000 of the prosthetic wrist 10.

The module of pronation-supination 1200 is preferably configured to perform pronation and supination movements in an angular range of $\pm 90°$ about the longitudinal axis V shown in Figure 9. Such an excursion may preferably be extended based on the application requirements by simply adjusting mechanical stops suitably arranged, or by operating an electronic control unit of the module 1200 itself, if present.

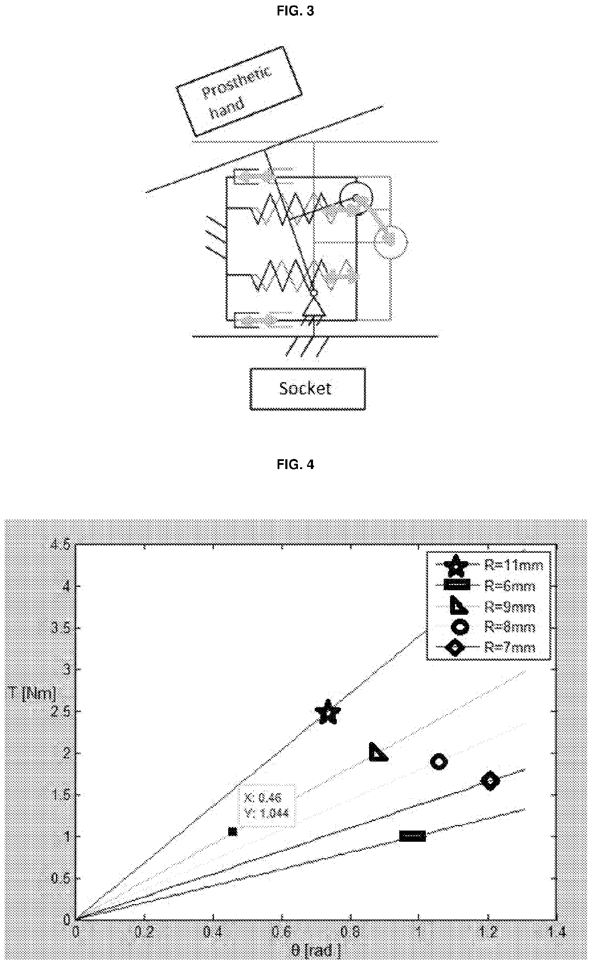

With reference to Figures 2 to 4 and 6 to 9, the module of flexion-extension 1000 according to the invention comprises a first proximal base 1, configured to connect in a fixed manner to the socket 100, and a second distal base 2, opposed to the first, configured to connect in a fixed manner to the prosthetic hand 101.

As anticipated, the distal base 2 is configured to connect to the prosthetic hand. When the prosthetic hand is subjected to flexion or extension, it moves by rotary motion.

According to a first aspect of the invention, the module of flexion-extension 1000 according to the present invention provides mechanical connection means 20 between said proximal base 1 and distal base 2 which comprises at least one Scotch-Yoke mechanism. A "Scotch-Yoke" mechanism is a mechanism configured to transform a rotary motion into a linear motion, and vice versa. In particular, the aforementioned at least one mechanism is arranged in the module 1000 to perform the transformation of the aforesaid rotary motion of the base 2 into a linear motion, as will be explained in further details below.

According to a preferred embodiment of the invention, the module of flexion-extension 1000 comprises two Scotch-Yoke mechanisms arranged in parallel. In particular, a first Scotch-Yoke mechanism is configured to perform the transformation of a rotary motion of the base 2 associated with a flexion, while a second Scotch-Yoke mechanism is configured to perform the transformation of a rotary motion of the base 2 associated with an extension.

Such Scotch-Yoke mechanisms are connected to elastic reaction means 40, which contrasts the rotation of the distal base 2 respectively in association with the flexion and extension movement, using stressing means 6.

In particular, each Scotch-Yoke mechanism is arranged in such a way as to move stressing means 6 of the aforementioned elastic reaction means 40 along a straight-line direction when the base 2 moves by extension or flexion. The stressing means 6 is configured to apply an elastic deformation force to the elastic reaction means 40. The elastic reaction means 40 is configured in such a way that, when the flexion/extension movement of the base 2 stops (i.e. when they are discharged and no longer subjected to external stresses), they exert an elastic return force on the Scotch-Yoke mechanisms such as to return the 1000 module to the rest configuration.

In order to describe into more detail the preferred configuration of the module 1000 subject-matter of the present discussion, the distal base 2 is connected to the first base 1 in such a way as to maintain a degree of freedom, which is expressed as the option to rotate with respect to a rotation axis P, the trace thereof is shown in Figure 2.

For this purpose, the distal base 2 is connected to the proximal base 1 preferably by means of a rotary coupling, realized for example by means of brackets protruding with respect to each of such bases 1, 2 and bearing seats configured to house a pin, such seats being preferably configured as through holes.

The brackets respectively connected to the first and second bases are coupled in such a way as to arrange the seats in an aligned configuration and to allow the insertion of pins inside thereof, so as to allow the mutual rotation of the proximal and distal bases 1, 2 about the main axis P of such pins. The axis P of the pins further coincides with the axis of the aforesaid seats - when the seats have a mutually aligned arrangement - and with the rotation axis of the distal base 2.

The aforementioned bases 1 and 2, which preferably have a plate configuration, are preferably coupled in such a way as to be specular with respect to the axis P. The bases 1 and 2 are preferably parallel at the rest configuration of the prosthetic hand (and of the module 1000).

Describing into even further detail, the proximal base 1 and distal base 2 are connected by mechanical moving means, for example comprising an elongated element or arm, preferably made of metallic material. Preferably, the module 1000 comprises two parallel arms, each of which is respectively connected to the proximal base 1 at an its own first proximal end, and to the distal base 2 at an its own second distal end.

The connection between each arm and the distal base 2 is rigid, and the arrangement is preferably such that each arm is substantially orthogonal with respect to the main surface of this distal base 2. The configuration is such that, when the prosthetic wrist 10 is in the rest condition, the axes of longitudinal development A, V - of the hand and of the socket, respectively - are parallel to the longitudinal development axis of each arm.

With specific reference to Figures 2 to 4, implementation and operation of a preferred embodiment of the module of flexion-extension 1000 according to the present invention will now be promptly described.

As anticipated, the relative flexion-extension movement realized between the proximal base 1 and the distal base 2 of the module 1000 is allowed by the presence of means suitable to allow the relative rotation between the aforesaid proximal base 1 and distal base 2, such as rotation guide means. In the preferred embodiment shown in the attached Figures, this rotation guide means comprises radial ball bearings (denoted by the numerical reference 3 in Figure 2). In particular, rotation guide means 3 is connected by means of, or is better mounted on, the mechanical connection elements interposed between the two bases 1, 2. Preferably, there is a radial ball bearing for each of the two arms.

Preferably, the module 1000 further comprises sliding means 5, respectively connected to the mechanical moving means and to the stressing means, preferably comprising a cross member 6. This latter cross member 6 realizes, preferably on its own inner surface, a linear guide 7 configured to allow the translation of said sliding means 5.

According to the preferred embodiment shown in the attached Figures, the sliding means 5 is configured as comprising an elongated body, shaped like an arm or rod, rigidly connected to the mechanical moving means at a first end and substantially arranged in an orthogonal direction with respect to the latter. The further second end of the elongated body of the sliding means, on the other hand, has a sliding element, configured to engage with the aforesaid cross member 6. This sliding element can be a skate or a rotary element (roller).

The sliding element, following the described semi-circular trajectory of the distal base 2 in a flexion-extension movement, in turn slides along the cross member 6, causing its displacement by means of the rigid coupling with the mechanical moving means. The cross member 6, being supported by the linear guide 7, is therefore forced to move in translation according to a single degree of freedom.

The cross member 6 is further connected to the elastic reaction means 40, in particular which may be implemented by means of four linear compression springs 4. The translational motion of the cross member 6 causes the springs 4 to compress. According to an alternative configuration, the elastic reaction means may be provided to comprise tension springs, which are subjected to an elongation with respect to their discharge configuration (at rest) when the cross member translates.

The aforementioned implementation of the connection mechanisms between the two bases 1, 2 realizes a preferred configuration of a Scotch-Yoke mechanism, suitable for performing the transformation of the rotary motion of the base 2 into a linear compression motion of the elastic reaction means 40.

In other words, the configuration of the elastic reaction means 40 is such that, when subjected to a compression stress, reduces its overall dimensions along its main development direction. Such compression and shortening of the reaction means 40 allows a rotation of said distal base 2 about the axis P.

As anticipated, these elastic reaction means 40 may preferably comprise a pack of linear compression springs. The elasticity of the reaction means ensures the module of flexion-extension 1000 to elastically return to the rest position when the application of the external stress (i.e. the stress that moves the prosthesis 101, together with the base 2, according to a flexion/extension movement) stops. Once the stress on the elastic reaction means 40 has stopped, this

6

is configured to return the cross member 6, and ultimately the entire module 1000, to the rest position (proximal lower base 1 and distal upper base 2 parallel to each other, and therefore axis main part of the prosthetic hand aligned with the axis of the wrist/arm) due to the elastic return force.

In further other words, the preferred embodiment of the module of flexion-extension 1000 according to the present invention comprises a block of linear springs, apt to react to a compression stress, which are compressed by means of two Scotch-Yoke mechanisms arranged in parallel and in a symmetrical manner, which allow the transformation of the flexion-extension rotary motion into a linear motion suitable for compressing the elastic reaction means, preferably along the longitudinal axis of the latter (still with particular reference to linear compression springs). The spring compression during flexion/extension ensures the subsequent elastic return of the module 1000, and therefore of the prosthetic hand 101, to the rest position.

In particular, the rotary motion associated with the flexion-extension movement of the artificial wrist according to the present invention can be approximated by the pure rotation of a hinge placed at the rotation centre of the wrist (i.e. at the rotary connection pins between the proximal bases 1 and distal bases 2), then transformed into a linear motion by interposing the two Scotch-Yoke mechanisms, which operate in parallel.

According to a preferred variant of the invention, the elastic reaction means 4 is subjected to pre-compression in order to ensure, in use, to maintain the horizontal position of the prosthetic hand in rest condition (i.e. with the longitudinal development axes A and V, respectively of the hand prosthetic 101 and of the socket 100, being aligned). According to a preferred variant of the invention, the two bases 1, 2 define corresponding main surfaces which are substantially parallel at this rest configuration.

In other words, according to the preferred variant illustrated in the attached Figures, the four linear springs 4, preferably all identical to each other, are pre-compressed in order to maintain the hand in a horizontal position when in the condition whereby the main axis of the hand A it is parallel to the ground.

The aforementioned module of flexion-extension 1000, suitably re-adjusted, may of course be further used to implement the flexion-extension of an ankle prosthesis, or to realize a robotic joint for rehabilitation or assistance machines.

A detailed description of the preferred implementation parameters of the invention will be provided below.

As concerns the implementation of the active module of pronation-supination 1200, a gear motor may be used which comprises: a Maxon EC 32 flat Ø32 mm brushless motor, 6 Watts of power, equipped with Hall sensors connected to a Maxon GP planetary reducer 22 C Ø22 mm, with a reduction ratio of 316:1. The actuator may further be equipped with a 3-channel Maxon MR encoder. Preferred features of the actuator are listed below:

- Output speed without load: 29.5 rpm

- Reduction ratio: 316: 1

- Maximum efficiency: 49%

- Nominal value of output torque: 1.5 Nm

- Output speed: 14 rpm

- Maximum radial load (10 mm from the flange): 55 N

- Actuator mass: 113 g

- Ltot = 72 mm

- Dmax = 35 mm

The relationship of the angular displacement θ between the main axis of the prosthetic wrist A and the axis of the vessel V with the linear displacement x of the springs is:

$$x(\theta) = (2 * R * \theta) / \pi$$

The elastic torque generated by each spring 4 during compression proves to be dependent on the angle θ based on the relation:

$$T = K + x(\theta) * R = (2 + K * \theta * R^2) / \pi$$

where R indicates the distance of the spring axis from the rotation axis P of the prosthetic wrist and K indicates the spring stiffness.

With reference to the preferred embodiment of the module of flexion-extension 1000, comprising four linear compression springs 4 arranged in parallel, the relationship of the elastic torque with the rotation 8 of the prosthetic wrist is:

$$T_{TOT} = 4 * T = 2 * (2 * K * \theta * R^2) / \pi$$

In order for the prosthetic wrist 10 to be able to maintain the hand 101 in a horizontal position when in condition of the main axis A of the hand being parallel to the ground, the springs require to be pre-compressed. The level of pre-compression will depend on the weight of the prosthetic hand to be connected to the wrist, on the position of its gravity centre with respect to the rotation centre of the wrist and on the distance R.

The overall path of the springs 4 is to be equal to the sum of the pre-compression value and the value required to ensure a wrist range of motion of about +/-75° (1,31 rad):

$$Path_{TOT} = x_{MAX} + x_p$$

With:

$$x_{MAX} = 2R * 1,31(rad) / \pi$$

By way of example, the sizing has been performed assuming a weight for the prosthetic hand equal to 640 grams. In order to maintain the prosthetic hand 101 in a horizontal position when the main axis A of the hand is parallel to the ground, the artificial joint according to the present invention is required to exert a torque T equal to about 1 Nm.

Across an iterative process aimed at comparing parameters of the artificial wrist and parameters of commercial springs, an artificial wrist joint has been sized for the aforementioned particular application. The process resulted in selecting a spring with a rigidity constant K equal to 11,77 N/mm, which seems the most suitable to fulfil the required specifications.

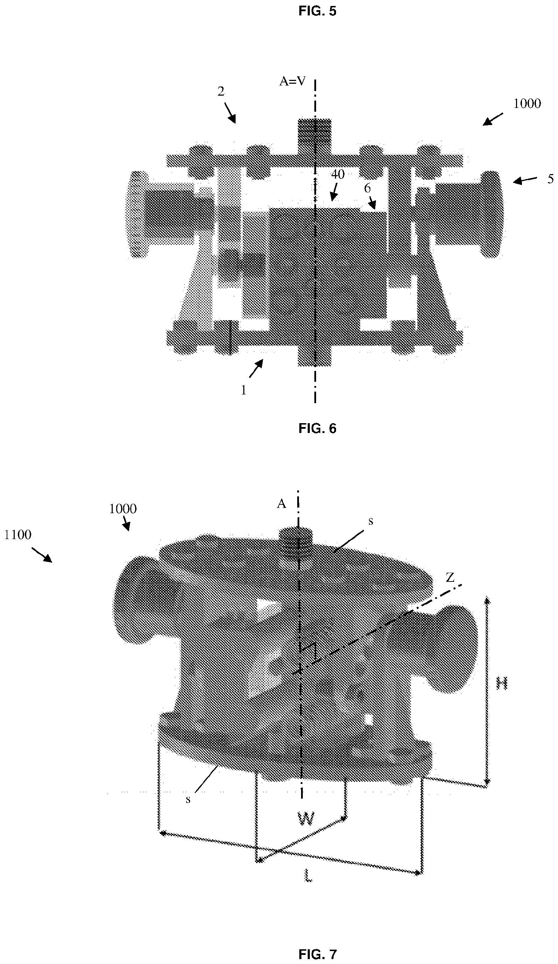

The steps of the iterative selection process will be listed below. Assuming a spring stiffness constant equal to 11,77 N/mm (commercial spring value), the value of the torque T generated by the prosthetic wrist 8 has been calculated and shown by the graph of Figure 5 as a function of the rotation angle, for different values of R.

It should be apparent that the spring pre-compression required to ensure maintaining the hand in a horizontal position in the configuration wherein the main axis A of the hand is parallel to the ground, depends on the value of R and the value of 8 such that a torque equal to about 1 Nm is obtained.

Since the value of R affects the dimension H of the wrist (Figure 7) along the direction A, while the pre-compression of the springs affects the total path of the spring and thence the dimension W of the wrist along the direction Z, a value of R equal to about 9 mm seems to achieve a right balance between overall dimension in the vertical direction of the wrist joint and pre-compression of the springs.

Based on the selected parameters, the pre-compression of the spring is calculated as follow:

$x_p$ = 2,63 mm

and the path of the spring aimed at ensuring a range of motion of +/-75° is equal to:
Xmax = 7,5 mm

which shows that the total path of the spring must be greater than:

$$Path_{TOT} = x_{MAX} + x_p = 7,5 + 2,63 = 10,13 \text{ mm}$$

Flexion/extension can reach approximately ±75°, as shown in Figures 8A to 8C. Furthermore, a preferred variant of the module of flexion-extension according to the present invention provides that five predetermined locking positions may be realized, every 25° (Figure 9).

Furthermore, the mechanical joint 1100 according to the invention is preferably provided with a blocking system 5, to ensure its use even in rigid mode.

In particular, the module 1000 comprises a blocking system 5 configured to allow the distal base 2 to be fixed in predetermined flexion-extension positions, wherein the axis A, which is an axis orthogonal to the distal base 2, has an inclination θ with respect to the longitudinal axis V between about -50° and about -25°, and about +25° and about +50°.

Preferably, the distal base 2 may be locked with respect to the proximal base 1 according to five different angular positions of the longitudinal axis A with respect to the axis V, which positions correspond to values of θ equal to approximately: - 50°, -25°, 0°, 25° and 50°. The angle θ equal to 0° corresponds to the rest configuration. The locking occurs due to the presence of selective locking means of the module of flexion-extension in the aforementioned predetermined angular configurations.

This locking means may comprise a mechanical locking mechanism, preferably comprising a first and a second spring piston, the frame of which is mounted on the arm perpendicular to the proximal base. In particular, the first and second spring pistons are arranged symmetrically in an external lateral position with respect to the module of flexion-extension.

The pistons are apt to prevent the rotation of the module by inserting into dedicated locking holes, which are arranged on the arms perpendicular to the distal base connected to the prosthetic hand. The holes are made in series along a curved trajectory, which follows the trajectory of rotation of the base distal to the proximal base.

The configuration is such that, when at least one small piston is inserted, it serves as an obstacle to the arm rotation, preventing the rotation of the distal base with respect to the proximal base. The pistons are selectively retractable in order to be brought into a configuration of non-interference with the locking holes, in such a way as to allow the free rotation of the distal base with respect to the proximal base, in order to realize the joint flexion-extension.

With reference to Figure 9, the preferred dimensions of the flexion-extension module according to the present invention are equal to 46,5 mm height measured along the axis A, 73 mm length of bases measured along the axis P and 40 mm width of the bases measured along the axis L. The overall weight of the preferred embodiment of the artificial joint according to the present invention, with details preferably made of aluminium class 6000, is equal to about 150 g.

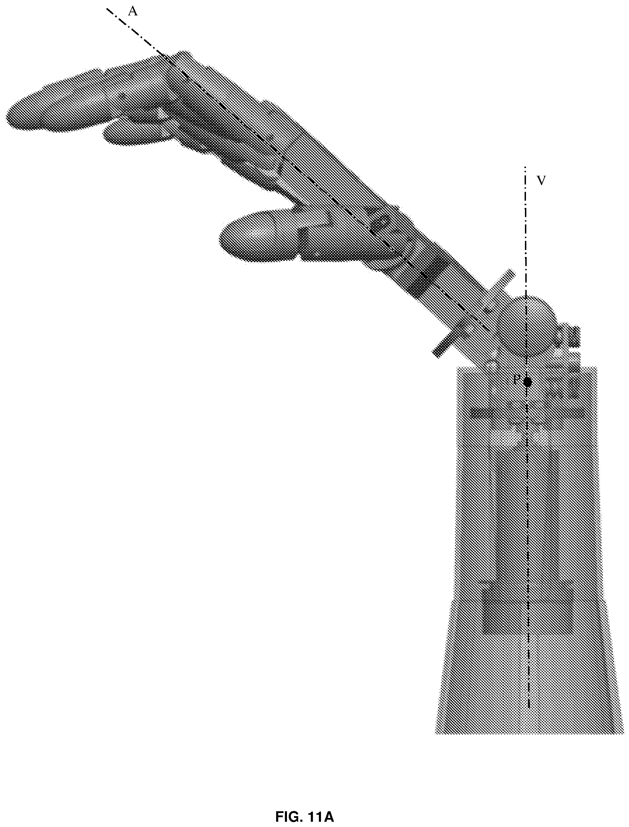

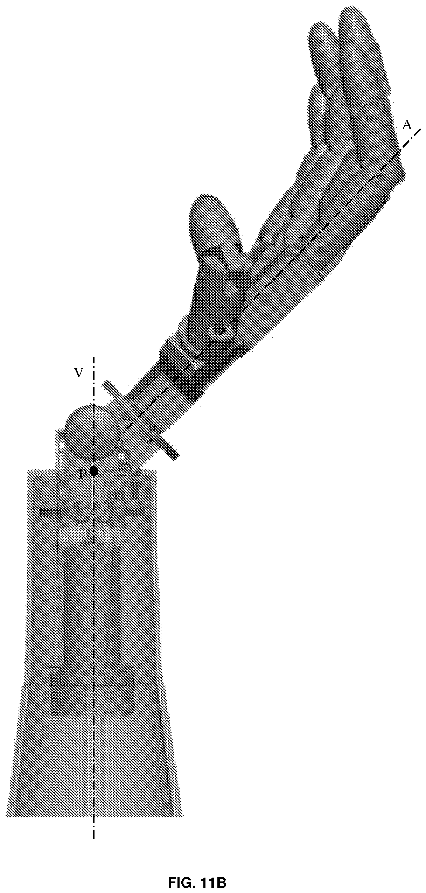

In Figures 10A to 11B an example of integration of the artificial joint of the invention (comprising the module of pronation-supination 1200 and the module of flexion-extension 1000) between a socket 100 and a prosthetic hand 101 is shown, wherein the anthropomorphism requirement is fulfilled.

In particular, the flexion-extension module is configured to operate according to two modes: flexible and rigid.

According to the flexible mode, the module is configured to automatically return to the rest position, i.e. without application of an external force needed, by simulating the non-in-contraction human joint. From the above resting position, the prosthetic wrist is displaced by the patient's contralateral healthy limb to perform flexion or extension. Such flexible mode allows the implementation of an adaptive prosthetic wrist, which promotes interaction with the surrounding environment.

On the other hand, operation according to the rigid mode allows to determine an orientation in the space of the prosthetic hand connected to the wrist, and to allow firm and safe grips for the manipulation of heavy objects. To implement such method, locking positions are preferably provided as described above, wherein the prosthetic wrist joint is in a rigid and stable configuration, providing the terminal organ with strength and resistance. In particular, a piston blocking system may be provided, as will be described in detail below.

As concerns the dimensional aspect, the preferred dimensions of the flexion-extension module according to the present invention are: height (measured along axis A) of between about 25 mm and 27 mm, width (measured along the axis P) is equal to about 67.8 mm, thickness (measured along the axis L, perpendicular to the axes A and P, again shown in Figure 9) of about 40 mm, while the value of mass can range from a minimum of about 55 g, to a maximum of about 89 g. As concern the active pronation-supination module, this is preferably configured to have an output torque of 2 Nm (i.e. apt to handle a weight of about 5 kg), a mass equal to about 150 g and an angular speed among about 30 and 60 rpm.

Due to the aforementioned preferred sizing parameters, the anthropometric dimensions are fulfilled. In such a way, it is possible to implement a wrist prosthesis by means of the joint of the invention which can be defined as anthropomorphic, and is particularly suitable for promoting the acceptance process thereof by the amputee, supporting him to overcome psychological traumas resulting from the traumatic event of the amputation.

The present invention benefits from a very wide applicability, which encompasses all the areas of robotics wherein the presence of a passive elastic joint is required: from automation and industrial robotics, wherein objects of various shapes and consistencies are grasped, moved, handled or assembled, to biomedical robotics, considering the recent continuous increasing number of robotic devices used in hospital environments (such as, in surgical and rehabilitation sectors). One example out of many is the application of assistance and rehabilitation systems for the upper and lower limbs, to assist the pronation-supination and flexion-extension movements of wrist and ankle. In the latter case, the pre-loaded elastic reaction means will compress during the walking *stance* phase and subsequently extend during the *swing* phase.

The use of the invention is further provided in further areas of robotics: service robotics, rescue robotics or even for exploration, with reference to complex or at risk for humans' situations or environments, where the use of systems capable of precision positioning, due to orientation controlled management, is crucial.

The present invention has been so far described referring to preferred embodiments. It should be understood that further embodiments which refer to the same inventive core, as defined by the scope of protection of the claims set forth below, are possible.

**Claims**

1. Module of flexion-extension (1000) for an artificial articulation (10), comprising:

   - a proximal base element (1), configured to connect to a prosthetic socket (100), the prosthetic socket (100) having a first longitudinal axis (V);
   - a distal base element (2), configured to connect to a prosthesis (101), the prosthesis (101) having a second longitudinal axis coinciding with an axis orthogonal (A) to said distal base element (2);
   - mechanical connection means (20) of said proximal base element (1) with said distal base element (2), configured in such a way as to allow rotation of said distal base element (2) with respect to said proximal base element (1) about a rotation axis (P) substantially orthogonal to the first longitudinal axis (V) and to the second longitudinal axis (V) in such a way as to realize a movement of flexion/extension of the prosthesis (101) with respect to the socket (100);
   - stressing means (6) connected to said mechanical connection means (20) and to respective elastic reaction means (40),
   said elastic reaction means (40) being configured to exert a reaction force apt to counteract the said rotation of said distal base element (2),

   wherein

   when said mechanical connection means (20) is not operated, said elastic reaction means (40) is configured to maintain said proximal base element (1) and said distal base element (2) in a rest configuration, wherein the first longitudinal axis (V) of the prosthetic socket (100) and the second longitudinal axis (A) of the prosthesis (101) are aligned, in particular coincident,
   **characterised in that**, said mechanical connection means (20) comprises at least one Scotch-Yoke mechanism configured to transform a rotary motion of said distal base element (2) with respect to said proximal base element (1 ) in a linear motion of said stressing means (6), the overall configuration of said module of flexion-extension (1000) being such that: when said mechanical connection means (20) is operated by said rotation of said distal base element (2) with respect to said proximal base element (1 ), said stressing means (6) is linearly moved to elastically deform said elastic reaction means (40).

2. Module of flexion-extension (1000) according to claim 1, wherein said mechanical connection means (20) comprises a first and a second Scotch-Yoke mechanism mutually arranged in parallel, said first and second Scotch-Yoke mechanisms being connected to respective stressing means (6),
   wherein said first Scotch-Yoke mechanism is configured to transform a rotary motion of said distal base element (2) associated with a flexion, and said second Scotch-Yoke mechanism is configured to transform a rotary motion of said distal base element (2) associated with an extension.

3. Module of flexion-extension (1000) according to claim 1 or 2, wherein said stressing means (6) is configured to elastically deform by compression said elastic reaction means (40).

4. Module of flexion-extension (1000) according to the preceding claim when depending upon claim 2, wherein said first and second Scotch-Yoke mechanisms are connected to respective stressing means (6) which in turn is connected to a respective couple of linear compression springs (4).

5. Module of flexion-extension (1000) according to any of the preceding claims, wherein said proximal base element (1) and said distal base element (2) have a substantially planar configuration, each bearing a main external surface (s) substantially planar, wherein said rotation axis (P) is substantially parallel to said main external surfaces (s), and wherein preferably, in said rest configuration, said longitudinal axis (V) and said orthogonal axis (A) are substantially coincident and orthogonal to said main external surfaces (s).

6. Module of flexion-extension (1000) according to any of the preceding claims, comprising a blocking system (5) of said distal base element (2) in predetermined positions of flexion/extension, wherein said orthogonal axis (A) has an inclination (θ) with respect to said longitudinal axis (V) comprised between about -50° and about -25°, and about +25°and about +50°.

7. Module of flexion-extension (1000) according to any of the preceding claims, wherein said elastic reaction means (40) is pre-compressed.

8. Mechanical joint (1100), apt to implement an artificial articulation, wherein the mechanical joint is configured to be connected to a prosthetic socket (100) at its own proximal end (a) and to a prosthesis (101) at its own distal end (b), the prosthetic socket (100) having a first longitudinal axis (V) and the prosthesis (101) having a second longitudinal axis (A),
**characterised in that** said mechanical joint (1100) comprises:

   - a module of flexion-extension (1000) according to any of the preceding claims; and
   - a module of pronation-supination (1200), apt to realize a rotary motion of the prosthesis (101) about an axis coinciding with the longitudinal axis (V), in such a way as to realize a pronation/supination movement of the prosthesis (101) with respect to the prosthetic socket (100).

9. Artificial articulation (10) comprising a mechanical joint (1100) according to claim 8, wherein the artificial articulation is an artificial wrist articulation.

10. Prosthesis assembly (1010), comprising an artificial articulation (10) according to claim 9, a prosthetic socket (100) and a prosthetic hand (101), wherein said artificial articulation (10) is connected respectively to said prosthetic socket (100) at its own proximal end (a) and to said prosthetic hand (101) at its own distal end (b).


**Patentansprüche**

1. Beugestreckmodul (1000) für ein künstliches Gelenk (10), umfassend

   - ein proximales Basiselement (1), das ausgebildet ist zum Verbinden mit einem Prothesenschaft (100), wobei der Prothesenschaft (100) eine erste longitudinale Achse (V) hat;
   - ein distales Basiselement (2), das ausgebildet ist zum Verbinden mit einer Prothese (101), wobei die Prothese (101) eine zweite longitudinale Achse hat, die mit einer Achse (A) zusammenfällt, welche orthogonal zu dem distalen Basiselement (2) ist;
   - mechanische Verbindungsmittel (20) des proximalen Basiselements (1) mit dem distalen Basiselement (2), die in einer Weise ausgebildet sind zum Erlauben eines Drehens des distalen Basiselements (2) bezüglich des proximalen Basiselements (1) um eine Rotationsachse (P), welche im Wesentlichen orthogonal zu der ersten longitudinalen Achse (V) und der zweiten longitudinalen Achse (V) ist, so dass eine Beuge-/Streckbewegung der Prothese (101) bezüglich des Schafts (100) realisiert wird;
   - Spannmittel (6), die mit den mechanischen Verbindungsmitteln (20) und jeweiligen elastischen Reaktionsmitteln (40) verbunden sind, wobei die elastischen Reaktionsmittel (40) so ausgestaltet sind, dass sie eine Reaktionskraft ausüben, um der Drehung des distalen Basiselements (2) entgegen zu wirken, wobei, wenn die mechanischen Verbindungsmittel (20) nicht betätigt werden, die elastischen Reaktionsmittel (40) so ausgestaltet sind, dass sie das proximale Basiselement (1) und das distale Basiselement (2) in einer Ruhekonfiguration halten, wobei die erste longitudinale Achse (V) des Prothesenschafts (100) und die zweite longitudinale Achse (A) der Prothese (101) ausgerichtet sind, insbesondere zusammenfallen,

   **dadurch gekennzeichnet, dass** die mechanischen Verbindungsmittel (20) wenigstens einen Scotch-Yoke-Mechanismus umfassen, der so ausgestaltet ist, dass er eine Drehbewegung des distalen Basiselements (2) bezüglich des proximalen Basiselements (1) in eine lineare Bewegung der Spannmittel (6) umwandelt, wobei die Gesamtausgestaltung des Beugestreckmoduls (1000) derart ist, dass: wenn die mechanischen Verbindungsmittel (20) durch die Drehung des distalen Basiselements (2) bezüglich des proximalen Basiselements (1) betätigt werden, die Spannmittel (6) linear bewegt werden, um die elastischen Reaktionsmittel (40) elastisch zu verformen.

2. Beugestreckmodul (1000) gemäß Anspruch 1, wobei die mechanischen Verbindungsmittel (20) einen ersten und einen zweiten Scotch-Yoke-Mechanismus umfassen, die wechselseitig parallel angeordnet sind, wobei der erste und der zweite Scotch-Yoke-Mechanismus mit jeweiligen Spannmitteln (6) verbunden sind,
wobei der erste Scotch-Yoke-Mechanismus so ausgestaltet ist, dass er eine Drehbewegung des distalen Basiselements (2) umwandelt, die mit einem Beugen assoziiert ist, und der zweite Scotch-Yoke-Mechanismus so ausgestaltet ist, dass er eine Drehbewegung des distalen Basiselements (2) umwandelt, die mit einem Strecken assoziiert ist.

3. Beugestreckmodul (1000) gemäß Anspruch 1 oder 2, wobei die Spannmittel (6) so ausgestaltet sind, dass sie die elastischen Reaktionsmittel (40) durch Kompression elastisch verformen.

4. Beugestreckmodul (1000) gemäß dem vorhergehenden Anspruch, wenn dieser von Anspruch 2 abhängt, wobei der erste und der zweite Scotch-Yoke-Mechanismus mit jeweiligen Spannmitteln (6) verbunden sind, welche wiederum jeweils mit einigen linearen Druckfedern (4) verbunden sind.

5. Beugestreckmodul (1000) gemäß einem der vorhergehenden Ansprüche, wobei das proximale Basiselement (1) und das distale Basiselement (2) eine im Wesentlichen planare Konfiguration haben, die jeweils eine im Wesentlichen ebene Hauptaußenfläche (s) hervorbringen, wobei die Drehachse (P) im Wesentlichen parallel zu den Hauptaußenflächen (s) ist, und wobei vorzugsweise in der Ruhekonfiguration die longitudinale Achse (V) und die orthogonale Achse (A) im Wesentlichen zusammenfallen und orthogonal zu den Hauptaußenflächen (s) sind.

6. Beugestreckmodul (1000) gemäß einem der vorhergehenden Ansprüche, umfassend ein Blockiersystem (5) des distalen Basiselements (2) in vorbestimmten Positionen des Beugens/Streckens, wobei die orthogonale Achse (A) bezüglich der longitudinalen Achse (V) eine Neigung (Θ) zwischen etwa -50° und etwa -25° und etwa +25° und etwa +50° umfasst.

7. Beugestreckmodul (1000) gemäß einem der vorhergehenden Ansprüche, wobei die elastischen Reaktionsmittel (40) vorkomprimiert sind.

8. Mechanische Verbindung (1100), geeignet zum Implementieren eines künstlichen Gelenks, wobei die mechanische Verbindung so ausgestaltet ist, dass sie an ihrem eigenen proximalen Ende (a) mit einem Prothesenschaft (100) und an ihrem eigenen distalen Ende (b) mit einer Prothese (101) verbunden wird, wobei der Prothesenschaft (100) eine erste longitudinale Achse (V) hat und die Prothese (101) eine zweite longitudinale Achse (A) hat, **dadurch gekennzeichnet, dass** die mechanische Verbindung (1100) umfasst:

- ein Beugestreckmodul (1000) gemäß einem der vorhergehenden Ansprüche; und
- ein Pronations-Supinations-Modul (1200), das geeignet ist, eine Drehbewegung der Prothese (101) um eine Achse zu realisieren, die mit der longitudinalen Achse (V) zusammenfällt, so dass eine Pronations-/Supinations-Bewegung der Prothese (101) bezüglich des Prothesenschafts (100) realisiert wird.

9. Künstliches Gelenk (10) umfassend eine mechanische Verbindung (1100) gemäß Anspruch 8, wobei das künstliche Gelenk ein künstliches Handgelenk ist.

10. Prothesenanordnung (1010) umfassend ein künstliches Gelenk (10) gemäß Anspruch 9, einen Prothesenschaft (100) und eine Prothesenhand (101), wobei das künstliche Gelenk (10) an seinem eigenen proximalen Ende (a) mit dem Prothesenschaft (100) verbunden ist und an seinem eigenen distalen Ende (b) mit der Prothesenhand (101) verbunden ist.

**Revendications**

1. Module de flexion-extension (1000) pour une articulation artificielle (10), comprenant :

un élément de base proximal (1) configuré pour se raccorder à une emboîture prothétique (100), l'emboîture prothétique (100) ayant un premier axe longitudinal (V) ;
un élément de base distal (2) configuré pour se raccorder à une prothèse (101), la prothèse (101) ayant un second axe longitudinal coïncidant avec un axe orthogonal (A) audit élément de base distal (2) ;
un moyen de raccordement mécanique (20) dudit élément de base proximal (1) avec ledit élément de base distal (2) configuré afin de permettre la rotation dudit élément de base distal (2) par rapport audit élément de base proximal (1) autour d'un axe de rotation (P) sensiblement orthogonal au premier axe longitudinal (V) et au second axe longitudinal (V) afin de réaliser un mouvement de flexion/extension de la prothèse (101) par rapport à l'emboîture (100) ;

un moyen de tension (6) raccordé audit moyen de raccordement mécanique (20) et au moyen de réaction élastique (40) respectif,

ledit moyen de réaction élastique (40) étant configuré pour exercer une force de réaction apte à s'opposer à ladite rotation dudit élément de base distal (2),

dans lequel lorsque ledit moyen de raccordement mécanique (20) n'est pas actionné, ledit moyen de réaction élastique (40) est configuré pour maintenir ledit élément de base proximal (1) et ledit élément de base distal (2) dans une configuration de repos, dans lequel le premier axe longitudinal (V) de l'emboîture prothétique (100) et le second axe longitudinal (A) de la prothèse (101) sont alignés, en particulier coïncident,

**caractérisé en ce que** ledit moyen de raccordement mécanique (20) comprend au moins un mécanisme à bielle et manivelle configuré pour transformer un mouvement rotatif dudit élément de base distal (2) par rapport audit élément de base proximal (1) en un mouvement linéaire dudit moyen de tension (6), la configuration globale dudit module de flexion-extension (1000) étant telle que : lorsque ledit moyen de raccordement mécanique (20) est actionné par ladite rotation dudit élément de base distal (2) par rapport audit élément de base proximal (1), ledit moyen de tension (6) est déplacé de manière linéaire pour déformer élastiquement ledit moyen de réaction élastique (40).

2. Module de flexion-extension (1000) selon la revendication 1, dans lequel ledit moyen de raccordement mécanique (20) comprend un premier et un second mécanisme à bielle et manivelle mutuellement agencés en parallèle, lesdits premier et second mécanismes à bielle et manivelle étant raccordés au moyen de tension (6) respectif,

dans lequel ledit premier mécanisme à bielle et manivelle est configuré pour transformer un mouvement rotatif dudit élément de base distal (2) associé à une flexion, et ledit second mécanisme à bielle et manivelle est configuré pour transformer un mouvement rotatif dudit élément de base distal (2) associé à une extension.

3. Module de flexion-extension (1000) selon la revendication 1 ou 2, dans lequel ledit moyen de tension (6) est configuré pour déformer élastiquement par compression, ledit moyen de réaction élastique (40).

4. Module de flexion-extension (1000) selon la revendication précédente lorsqu'elle dépend de la revendication 2, dans lequel lesdits premier et second mécanismes à bielle et manivelle sont raccordés au moyen de tension (6) respectif qui est raccordé, à son tour, à une paire respective de ressorts de compression linéaires (4).

5. Module de flexion-extension (1000) selon l'une quelconque des revendications précédentes, dans lequel ledit élément de base proximal (1) et ledit élément de base distal (2) ont une configuration sensiblement planaire, chacun supportant une surface externe principale (s) sensiblement planaire, dans lequel ledit axe de rotation (P) est sensiblement parallèle auxdites surfaces externes principales (s), et dans lequel, de préférence, dans ladite configuration de repos, ledit axe longitudinal (V) et ledit axe orthogonal (A) coïncident sensiblement et sont orthogonaux auxdites surfaces externes principales (s).

6. Module de flexion-extension (1000) selon l'une quelconque des revendications précédentes, comprenant un système de blocage (5) dudit élément de base distal (2) dans des positions prédéterminées de flexion/extension, dans lequel ledit axe orthogonal (A) a une inclinaison (θ) par rapport audit axe longitudinal (V) comprise entre environ -50° et environ -25°, et environ +25° et environ +50°.

7. Module de flexion-extension (1000) selon l'une quelconque des revendications précédentes, dans lequel ledit moyen de réaction élastique (40) est pré-comprimé.

8. Articulation mécanique (1100), apte à mettre en oeuvre une articulation artificielle, dans lequel l'articulation mécanique est configurée pour être raccordée à une emboîture prothétique (100) au niveau de sa propre extrémité proximale (a) et à une prothèse (101) au niveau de sa propre extrémité distale (b), l'emboîture prothétique (100) ayant un premier axe longitudinal (V) et la prothèse (101) ayant un second axe longitudinal (A), **caractérisé en ce que** ladite articulation mécanique (1100) comprend :

un module de flexion/extension (1000) selon l'une quelconque des revendications précédentes ; et
un module de pronation-supination (1200) apte à réaliser un mouvement de rotation de la prothèse (101) autour d'un axe coïncidant avec l'axe longitudinal (V), afin de réaliser un mouvement de pronation-supination de la prothèse (101) par rapport à l'emboîture prothétique (100).

9. Articulation artificielle (10) comprenant une articulation mécanique (1100) selon la revendication 8, dans laquelle l'articulation artificielle est une articulation de poignet artificielle.

10. Ensemble prothétique (1010) comprenant une articulation artificielle (10) selon la revendication 9, une emboîture prothétique (100) et une main prothétique (101), dans lequel ladite articulation artificielle (10) est respectivement raccordée à ladite emboîture prothétique (100) au niveau de sa propre extrémité proximale (a) et à ladite main prothétique (101) au niveau de sa propre extrémité distale (b).

## FIG. 1

a  Prosthetic wrist  b

100
Socket

1200
Module of
pronation-
supination

10

1000
Module of
flexion-
extension

101
Prosthetic
hand

## FIG. 2

**FIG. 3**

**FIG. 4**

**FIG. 5**

**FIG. 6**

**FIG. 7**

V

V = A

A

FIG. 8A

FIG. 8B

V

A

FIG. 8C

A

V

θ

P

L

FIG. 9

FIG. 10A

FIG. 10B

FIG. 10C

FIG. 11A

FIG. 11B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007173955 A1